# EUROPÄISCHE PATENTSCHRIFT

(11) **EP 3 016 928 B1**
(45) Veröffentlichungstag und Bekanntmachung des Hinweises auf die Patenterteilung: **09.08.2017**
(21) Anmeldenummer: 14733613.5
(22) Anmeldetag: 25.06.2014
(51) Int. Cl.: C07C 46/08, C07C 50/02

(54) **VERFAHREN ZUR HERSTELLUNG VON 2,3,5-TRIMETHYLBENZOCHINON DURCH OXIDATION VON 2,3,6-TRIMETHYLPHENOL**
METHOD FOR PRODUCING 2,3,5-TRIMETHYL BENZOQUINONE BY OXIDATION OF 2,3,6-TRIMETHYLPHENOL
PROCÉDÉ DE PRODUCTION DE 2,3,5-TRIMÉTHYLBENZOQUINONE PAR OXYDATION DE 2,3,6-TRIMÉTHYLPHÉNOL

(30) Priorität: 02.07.2013 EP 13174688; 02.07.2013 US 201361841946 P
(43) Veröffentlichungstag der Anmeldung: 11.05.2016
(60) Teilanmeldung: 16187815.2
(73) Patentinhaber: BASF SE, 67056 Ludwigshafen am Rhein (DE)
(72) Erfinder: DEHN, Richard, 67061 Ludwigshafen (DE); KRAUS, Michael, 67227 Frankenthal (DE); DEHN, Martine, 67061 Ludwigshafen (DE); DANZ, Manuel, 68723 Plankstadt (DE); TELES, Joaquim Henrique, 67165 Waldsee (DE)
(74) Vertreter: BASF IP Association
(86) Internationale Anmeldenummer: PCT/EP2014/063425
(87) Internationale Veröffentlichungsnummer: WO 2015/000767

(56) Entgegenhaltungen:
- EP-A1- 0 369 823
- EP-A2- 0 475 272

## Beschreibung

Gegenstand der vorliegenden Erfindung ist ein Verfahren zur Herstellung von 2,3,5-Trimethylbenzochinon oder einer Mischung enthaltend 2,3,5-Trimethylbenzochinon umfassend den folgenden Schritt: Oxidation von 2,3,6-Trimethylphenol mit Sauerstoff oder einem sauerstoffhaltigen Gas in einem zwei- oder mehrphasigen Reaktionsmedium in Gegenwart eines Katalysators oder Katalysatorsystems mindestens enthaltend ein Kupfer(II)-halogenid zu einer Mischung enthaltend 2,3,5-Trimethylbenzochinon, dadurch gekennzeichnet, dass das Reaktionsmedium Wasser und mindestens einen sekundären aliphatischen azyklischen Alkohol mit 6 oder mehr, vorzugsweise 7 oder mehr, Kohlenstoffatomen enthält.

Hierin beschrieben ist auch eine Mischung enthaltend 2,3,5-Trimethylbenzochinon, wobei die Mischung herstellbar oder hergestellt ist durch das erfindungsgemäße Verfahren.

Außerdem ist hierin die Verwendung eines sekundären aliphatischen azyklischen Alkohols mit 6 oder mehr, vorzugsweise 7 oder mehr Kohlenstoffatomen als Lösungsmittel bei der Oxidation von 2,3,6-Trimethylphenol zu 2,3,5-Trimethylbenzochinon beschrieben.

Darüber hinaus wird auch die Verwendung des nach dem erfindungsgemäßen Verfahren hergestellten 2,3,5-Trimethylbenzochinon oder einer nach dem erfindungsgemäßen Verfahren hergestellten Mischung enthaltend 2,3,5-Trimethylbenzochinon bei der Synthese von Vitamin E, insbesondere zur Herstellung von 2,3,6-Trimethylhydrochinon beschrieben.

2,3,6-Trimethylbenzochinon (**1**) ist als Vorprodukt für die industrielle Synthese von α-Tocopherol = Vitamin E (**3**) von großer Bedeutung. Hierzu wird (1) zunächst zu 2,3,6-Trimethylhydrochinon (**2**) katalytisch hydriert und anschließend unter Verwendung einer Lewis-Säure als Katalysator mit Isophytol kondensiert, um (**3**) zu bilden (Schema 1).

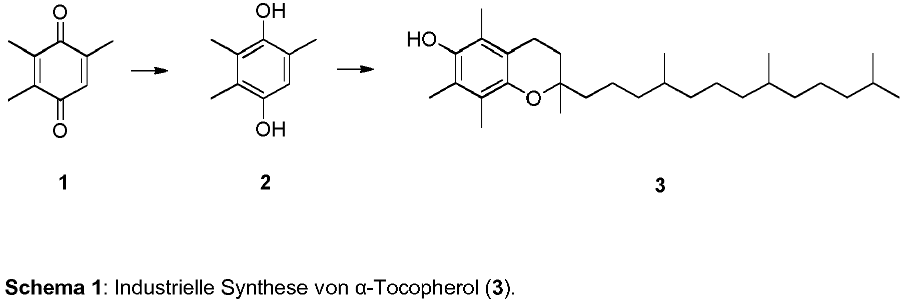

Für die Synthese von (**1**) sind eine Reihe technisch realisierter Synthesen bekannt. Fast alle aus dem Stand der Technik bekannten Verfahren verwenden zur Synthese von (**1**) die Oxidation von 2,3,6-Trimethylphenol (**4**), das wiederum auf verschiedenen Wegen erhalten werden kann. Für die Oxidation von (**4**) zu (**1**) wird bevorzugt Sauerstoff als preiswertes Oxidationsmittel verwendet (Schema 2).

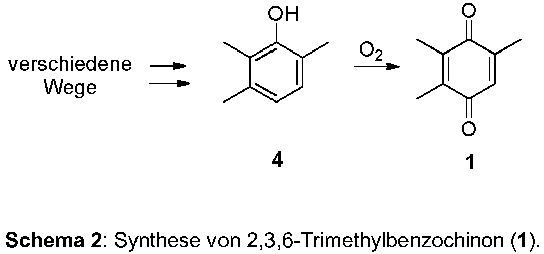

Die effizientesten Verfahren zur Oxidation von (**4**) zu (**1**) verwenden Kupfer(II)halogenide oder Kupfer(II)halogenkomplexe als Katalysatoren. Diese Verfahren sind aber auch mit Problemen behaftet. Zu nennen sind zum Beispiel eine aufwändige Rückführung des verwendeten Lösungsmittels und/oder des verwendeten Katalysators und vor allem die Bildung von unerwünschten Nebenprodukten. Besonders hervorzuheben ist die Bildung von halogenierten, i. d. R. chlorierten Nebenprodukten, die während der Aufarbeitung unter Freisetzung von HCl zerfallen und so zu Korrosion und zu Verlusten des Wertprodukts (**1**) führen.

DE 2 221 624 beschreibt die Oxidation von 2,3,6-Trimethylphenol mit Sauerstoff in Anwesenheit von Kupferhalogeniden, vornehmlich Kupfer(II)chlorid-Dihydrat, in polaren, wasserlöslichen oder unbeschränkt mit Wasser mischbaren Lösungsmitteln, bevorzugt Dimethylformamid. Dieses Verfahren hat aber den Nachteil, dass es schwierig ist, aus dem Reaktionsgemisch das Produkt zu isolieren und den Katalysator zurückzuführen.

EP 127 888 beschreibt die Verwendung von Kupfer(II)-Halogenidkomplexen mit der allgemeinen Formel M_{ℓ}[Cu(II)ₘXₙ]ₚ, wie zum Beispiel Li[CuCl₃] oder K₂[CuCl₄], als Katalysatoren für die Oxidation von 2,3,6-Trimethylphenol mit Sauerstoff in einem Gemisch aus Wasser und einem aliphatischen Alkohol mit 4 bis 10 Kohlenstoffatomen als Lösungsmittel. Da das Lösungsmittel eine Mischungslücke mit Wasser aufweist, findet die Reaktion in einem Gemisch aus zwei flüssigen Phasen statt. Dadurch erreicht man hohe Reaktionsraten, und der Katalysator lässt sich leicht als wässrige Lösung durch eine Phasentrennung zurückführen. Die eingesetzten aliphatischen Alkohole können 4 bis 10 Kohlenstoffatome, bevorzugt 5 bis 10 Kohlenstoffatome enthalten. Als bevorzugte Lösungsmittel werden primäre Alkohole genannt, bspw. n-Butanol, n-Amylalkohol, Isoamylalkohol, n-Hexanol, 2-Ethylhexanol, n-Heptanol, n-Octanol, n-Nonanol und n-Decanol.

EP 167 153 beschreibt die Verwendung desselben Katalysators wie in EP 127 888 in einem Gemisch aus Wasser und aliphatischen Alkoholen mit 5 bis 10 Kohlenstoffatomen als Lösungsmittel. Bevorzugt werden primäre Alkohole, bspw. n-Amylalkohol, Isoamylalkohol, n-Hexanol, 2-Ethylhexanol, n-Heptanol, n-Octanol, n-Nonanol und n-Decanol genannt. Die Reaktion wird in "semibatch"-Fahrweise durchgeführt, wodurch weniger Nebenprodukte gebildet werden Die Reaktion lässt sich ferner leichter kontrollieren, und es wird weniger Katalysator benötigt.

EP 294 584 beschreibt die Verwendung eines Gemisches aus Kupfer(II)chlorid und Lithiumchlorid als Katalysator und als Lösungsmittel ein wässriges Gemisch aus einem aromatischen Kohlenwasserstoff und einem aliphatischen Alkohol mit 1 bis 4 Kohlenstoffatomen. Beispielhaft genannt werden die primären Alkohole Methanol, Ethanol, n-Propanol, Isopropanol, n-Butanol, 2-Butanol und tert-Butanol. Zwar werden auch einige sekundäre und tertiäre Alkohole genannt, allerdings nur in Kombination mit einem aromatischen Kohlenwasserstoff. Darüber hinaus weisen diese Alkohole maximal 4 Kohlenstoffatome auf. Der Vergleich der Beispiele 9 und 11 der EP 294 584 zeigt außerdem, dass die Verwendung von Isopropanol als sekundärem Alkohol zu niedrigeren Ausbeuten führt als die Verwendung von n-Propanol (91,6 vs. 94.1% unter ansonsten identischen Bedingungen). Darüber hinaus führt die Verwendung eines Lösungsmittelgemisches zu einer deutlich verkomplizierten Lösungsmittelrückführung.

EP 369 823 beschreibt die Verwendung von Kupfer(II)chlorid in Kombination mit bestimmten Stickstoffverbindungen (Hydroxylammoniumsalze, Oxime oder Ammoniumsalze) als Katalysator. Als Lösungsmittel werden bevorzugt aliphatische Alkohole mit 3 bis 10 Kohlenstoffatomen verwendet, wobei besonders bevorzugt verzweigte Alkohole mit 3 bis 6 Kohlestoffatome und ganz besonders bevorzugt tertiäre Alkohole, wie tert-Butanol oder tert-Amylalkohol, eingesetzt werden. Allerdings liegen die besten hier erzielten Ausbeuten an (**1**) (Beispiel 55: 94,5% mit tert-Amylalkohol und 95% mit tert-Butanol) bestenfalls auf demselben Niveau wie in älteren Schriften des Standes der Technik beschrieben, die den Zusatz von Stickstoffverbindungen nicht benötigen

M. Shimizu et al. (Bull. Chem. Soc. Jpn. 65 (1992) 1522) offenbaren ein Verfahren zur Oxidation von (**4**) unter Verwendung von Gemischen aus Cu(II)chlorid und Hydrochloriden verschiedener Amine, Hydroxylamin oder Oximen als Katalysatorsystem. Auch hier werden als Lösungsmittel neben primären Alkoholen sekundäre Alkohole, wie Isopropanol, sek-Butanol, 2-Pentanol und 3-Pentanol, tert-Butanol und tert-Amylalkohol verwendet, ohne dass ein besonderen Vorteil beim Einsatz von sekundären Alkoholen ersichtlich wird. Die bevorzugte Verwendung von Hydroxylamin als Zusatz, welcher bei der Oxidation verbraucht wird, macht dieses Verfahren unattraktiv.

EP 475 272 beschreibt die Verwendung von Mischungen aus Kupfer(II)chlorid und Erdalkalimetallchloriden, insbesondere MgCl₂, als Katalysator. Als Lösungsmittel werden gesättigte aliphatische Alkohole mit 5 bis 10 Kohlenstoffatomen aufgeführt. Als besonders bevorzugt werden 1-Pentanol, 1-Hexanol, 1-Heptanol, 1-Octanol, 1-Nonanol, 1-Decanol, 2-Ethyl-1-hexanol und Cyclohexanol genannt, d.h. mit Ausnahme von Cyclohexanol primäre Alkohole.

EP 1 092 701 beschreibt die Verwendung von Mischungen aus Kupfer(II)chlorid und anderen Metallchloriden aus der Gruppe Fe, Cr, Mn, Co, Ni, Zn oder seltenen Erden als Katalysator. Als Lösungsmittel werden verzweigte und unverzweigte aliphatische Alkohole mit 5 bis 10 Kohlenstoffatomen aufgeführt. Als besonders bevorzugt werden 1-Pentanol, 1-Hexanol, 1-Heptanol, 1-Octanol, 1-Nonanol, 1-Decanol, 2-Ethyl-1-hexanol und Cyclohexanol genannt, d.h. mit Ausnahme von Cyclohexanol primäre Alkohole.

JP 55 072 136 beschreibt die Verwendung von Kupfer(II)chlorid-Dihydrat als Katalysator in Polyethylenglykolen, wie zum Beispiel CH₃O(CH₂CH₂O)₉CH₃, als Lösungsmittel. Das Reaktionsgemisch kann wässrig aufgearbeitet werden, um den Katalysator zurückzuführen. Das gewünschte Produkt wird destillativ als Leichtsieder abgetrennt. Dieses Verfahren hat jedoch den Nachteil, dass sich schwersiedende Nebenprodukten im Lösungsmittel aufkonzentrieren und nur schwer zu entfernen sind.

So wie alle auf Cu-Halogeniden als Katalysatoren basierende Verfahren haben auch die zuvor beschriebenen Verfahren grundsätzliche Nachteile, die von der Bildung chlororganischer Nebenprodukte herrühren. Diese Nebenprodukte entstehen während der Durchführung der Oxidationsreaktion und resultieren aus der Chlorierung des Edukts (**4**), des Produkts (**1**) und ggf. des Lösungsmittels 1-Hexanol. Abbildung 1 zeigt einige typische Nebenprodukte, ohne jedoch eine vollständige Auflistung zu geben.

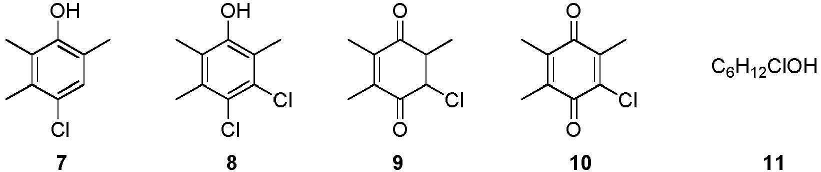

**Abbildung 1**: Typische Nebenprodukte bei der Oxidation von 2,3,6-Trimethylphenol (**4**) zu 2,3,6-Trimethylbenzochinon (**1**).

Die auftretenden Chlorierungsreaktionen bewirken einen direkten Selektivitätsverlust in der gewünschten Oxidationsreaktion und darüber hinaus ggf. einen Lösungsmittelverlust, der durch Zugabe von frischem Lösungsmittel ausgeglichen werden muss. Gleichzeitig verarmt die Katalysatorphase an Chlorid, weshalb die Katalysatorphase zur Regeneration in regelmäßigen Abständen mit Salzsäure behandelt werden muss.

Die chlororganischen Nebenprodukte spalten darüber hinaus - insbesondere bei thermischer Belastung - Chlorwasserstoff ab, der in den entsprechenden Anlagenteilen (z.B. in Destillationskolonnen, in denen die Reaktionsmischung erwärmt wird) zu erheblichen Korrosionsproblemen führt, weshalb teure Spezialstähle für nahezu alle Bereiche der Anlage verwendet werden müssen. Ferner induziert Chlorwasserstoff Zersetzungsreaktionen des Wertproduktes (**1**), was zu Ausbeuteverlusten, insbesondere im Sumpf der Destillationskolonne, führt. Die chlororganischen Nebenprodukte (z. B. die in Abb. 1 dargestellten Verbindungen (**7**) - (**11**)) haben i. d. R. Siedepunkte, die dem des Lösungsmittels oder dem des Produktes ähnlich sind, was die destillative Trennung des Reaktionsgemisches erschwert und sowohl Produkt- als auch Lösungsmittelverlust in Zwischensiederfraktionen bedingt. Chlorhaltige Verunreinigungen vergiften auch den Hydrierkatalysator der sich anschließenden Umsetzung des Chinons (**1**) zum Hydrochinon (**2**).

Zur Vermeidung dieser Nachteile werden im allgemeinen zusätzliche Aufarbeitungsschritte durchgeführt. EP 0 216 351 offenbart ein Konzept zur Abtrennung chlororganischer Nebenprodukte aus Rohausträgen der CuCl₂-vermittelten Oxidation von (**4**) zu (**1**). Die Abreicherung der Nebenprodukte erfolgt hier durch eine Basenwäsche. Die Basenwäsche ist aber nicht besonders effektiv und bedeutet immer einen Kompromiss zwischen der Reduzierung chlororganischer Verbindungen und Produktverlusten. Insgesamt ermöglicht diese Maßnahme zwar eine technische Umsetzung des Verfahrens gemäß Schema 3, jedoch führt die Basenwäsche durch den zusätzlichen Verfahrensschritt zu höheren Investitions- und Produktionskosten, zu Ausbeuteverlusten und somit insgesamt nur zu einer Milderung des hier beschriebenen Problems.

Eine primäre Aufgabe der vorliegenden Erfindung ist daher die Bereitstellung eines Verfahrens zur Herstellung von 2,3,5-Trimethylbenzochinon oder einer Mischung enthaltend 2,3,5-Trimethylbenzochinon durch Oxidation von 2,3,6-Trimethylphenol in Gegenwart eines Katalysators oder Katalysatorsystems mindestens enthaltend ein Kupfer(II)-halogenid, das alle Vorteile der aus dem Stand der Technik bekannten Verfahren aufweist, aber die Bildung der unerwünschten chlorierten Nebenprodukte minimiert.

Eine bevorzugte Aufgabe der vorliegenden Erfindung ist die Bereitstellung eines Verfahrens zur Herstellung von 2,3,5-Trimethylbenzochinon oder einer Mischung enthaltend 2,3,5-Trimethylbenzochinon durch Oxidation von 2,3,6-Trimethylphenol in Gegenwart eines Katalysators oder Katalysatorsystems mindestens enthaltend ein Kupfer(II)-halogenid, welches die Bildung chlorierter Nebenprodukte weitestgehend vermeidet, um auf diese Weise die Selektivität der Reaktion zu verbessern, den Lösungsmittelverlust durch Chlorierung des Lösungsmittels zu minimieren und ggf. die Notwendigkeit zusätzlicher Aufarbeitungsschritte sowie die Verwendung von Spezialwerkstoffen zu vermeiden.

Es wurde nun überraschend gefunden, dass durch die Verwendung eines sekundären aliphatischen azyklischen Alkohols mit 6 oder mehr, vorzugsweise 7 oder mehr Kohlenstoffatomen als Lösungsmittel bei der Oxidation von 2,3,6-Trimethylphenol in Gegenwart eines Katalysators oder Katalysatorsystems mindestens enthaltend ein Kupfer(II)-halogenid die Bildung von chlorierten Nebenprodukten unter gleichzeitiger Beibehaltung aller aus dem Stand der Technik bekannten Vorteile stark unterdrückt werden kann. Gegenstand der vorliegenden Erfindung ist somit ein Verfahren zur Herstellung von 2,3,5-Trimethylbenzochinon oder einer Mischung enthaltend 2,3,5-Trimethylbenzochinon umfassend den folgenden Schritt: Oxidation von 2,3,6-Trimethylphenol mit Sauerstoff oder einem sauerstoffhaltigen Gas in einem zwei- oder mehrphasigen Reaktionsmedium in Gegenwart eines Katalysators oder Katalysatorsystems mindestens enthaltend ein Kupfer(II)-halogenid zu einer Mischung enthaltend 2,3,5-Trimethylbenzochinon, dadurch gekennzeichnet, dass das Reaktionsmedium Wasser und mindestens einen sekundären aliphatischen azyklischen Alkohol mit 6 oder mehr, vorzugsweise 7 oder mehr, Kohlenstoffatomen enthält.

Bei dem erfindungsgemäßen Verfahren kann auf die Verwendung von Spezialstählen sowie auf eine Basenwäsche verzichtet werden. Das Rohprodukt ist unter thermischer Belastung während der Reindestillation deutlich stabiler, wodurch Ausbeuteverluste minimiert werden und gleichzeitig ein reineres und damit hochwertigeres Produkt erhalten wird.

Prinzipiell eignen sich für das erfindungsgemäße Verfahren alle aliphatischen azyklischen Alkohole, die 6 oder mehr, vorzugsweise 7 oder mehr, Kohlenstoffatome enthalten. Besonders bevorzugt ist die Verwendung von 3-Heptanol.

In einer vorteilhaften Ausgestaltung des erfindungsgemäßen Verfahrenes wird die Oxidation von 2,3,6-Trimethylphenol (**4**) mit einem sauerstoffhaltigen Stickstoffgas in einem zweiphasigen Reaktionsmedium in Gegenwart substöchiometrischer Mengen eines CuCl₂-Katalysators und stöchiometrischer Mengen von LiCl durchgeführt (Schema 3).

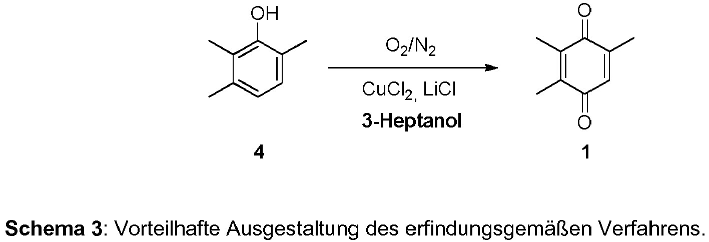

Bei dem erfindungsgemäßen Verfahren wird die Bildung chlororgansicher Nebenprodukte in der Oxidationsreaktion von 2,3,6-Trimethylphenol (**4**) zu 2,3,5-Trimethylbenzochinon (**1**) deutlich verringert.

Neben der signifikanten Reduktion der chlororganischen Nebenprodukte werden in Austrägen der in sekundären Alkoholen durchgeführten Oxidationsreaktion deutlich verringerte Mengen an Metallionen bzw. Chloridionen sowie deutlich weniger Wasser gefunden. Dies erleichtert die Extraktion zur Rückgewinnung des in der organischen Phase gelösten Katalysators. Da die Phasentrennung während der wässrigen Aufarbeitung deutlich schneller erfolgt, verkürzt und vereinfacht sich das Aufarbeitungskonzept im Vergleich zur Verwendung primärer Alkohole als Lösungsmittel erheblich. Da zudem die Wasserlöslichkeit sekundärer Alkohole gegenüber primären Alkoholen gleicher Kohlenstoffanzahl geringer ist, reduziert sich auch der Lösungsmittelverlust während der Extraktion.

In der oben beschriebenen vorteilhaften Ausgestaltung des erfindungsgemäßen Verfahrens besteht die Reaktionsmischung aus einer unteren wässrigen Katalysatorphase und einer oberen organischen Phase, die Lösungsmittel, Substrat und Reaktionsprodukte enthält. In diese zweiphasige Mischung wird unter Rühren ein Sauerstoff/Stickstoff-Strom eingespeist. Das Verfahren erfolgt vorzugsweise diskontinuierlich.

In einer vorteilhaften Ausgestaltung des erfindungsgemäßen Verfahrens wird die Mischung enthaltend 2,3,5-Trimethylbenzochinon in einem Schritt (ii) mit einer wässrigen alkalischen Lösung gewaschen.

Darüber hinaus ist es erfindungsgemäß bevorzugt, dass die Oxidation bei einer Temperatur zwischen 50 °C und 65 °C, vorzugsweise bei einer Temperatur zwischen 53 und 58 °C durchgeführt wird.

Es ist weiterhin erfindungsgemäß bevorzugt, dass die Oxidation über einen Zeitraum von 4 bis 8 Stunden, vorzugsweise über einen Zeitraum von 5-7 Stunden, durchgeführt wird.

In einer vorteilhaften Ausgestaltung des erfindungsgemäßen Verfahrens wird das Reaktionsmedium nach erfolgter Oxidation in einem Schritt (iii) einer Phasentrennung unterworfen und die organische Phase zur Rückgewinnung des in der organischen Phase gelösten Katalysators extrahiert.

Hierin beschrieben ist auch eine Mischung enthaltend 2,3,5-Trimethylbenzochinon, wobei die Mischung herstellbar oder hergestellt ist durch das erfindungsgemäße Verfahren. Durch das erfindungsgemäße Verfahren kann eine Mischung enthaltend 2,3,5-Trimethylbenzochinon erhalten werden, die einen Chlorgehalt von weniger als 0.5 g/100g und/oder einen Lithiumgehalt von weniger als 0.3 g/100g und/oder einen Kupfergehalt von weniger als 240 mg/kg aufweist.

Hierin beschrieben ist außerdem die Verwendung eines sekundären aliphatischen azyklischen Alkohols mit 6 oder mehr, vorzugsweise 7 oder mehr Kohlenstoffatomen als Lösungsmittel bei der Oxidation von 2,3,6-Trimethylphenol zu 2,3,5-Trimethylbenzochinon.

Darüber hinaus ist hierin die Verwendung des nach dem erfindungsgemäßen Verfahren hergestellten 2,3,5-Trimethylbenzochinon oder einer nach dem erfindungsgemäßen Verfahren hergestellten Mischung enthaltend 2,3,5-Trimethylbenzochinon bei der Synthese von Vitamin E, insbesondere zur Herstellung von 2,3,6-Trimethylhydrochinon beschrieben.

Die Erfindung wird anhand der nachfolgenden Beispiele näher erläutert.

### Analytik:

Am Ende der beispielhaften Versuche wurden die Phasen getrennt, separat gewogen, und die organischen Phasen wurden analysiert. Die quantitative Bestimmung des Gehaltes von (**1**) und (**4**) in den organischen Phasen erfolgte gaschromatographisch. Die Bestimmung des Gesamt-Chlorgehaltes erfolgte durch Elementaranalyse, und der Gehalt an Chloridionen wurde durch potentiometrische Titration mit einer Silbernitratlösung bestimmt. Die Differenz der zwei Werte ergibt den Gehalt an organisch gebundenem Chlor. Die quantitative Bestimmung von Kupfer und Lithium erfolgte durch Atomemissionsspektroskopie (ICP-OES).

### Beispiele 1 bis 10:

Ein 4 L-Stahlreaktor wird mit 657 g eines wässrigen Reaktionsmediums bestehend aus 151 g CuCl₂·2H₂O, 150 g LiCl und 365 g Wasser sowie 818 g des als Lösungsmittel dienenden Alkohols beschickt. Unter Rühren wird diese zweiphasige Mischung auf die gewünschte Anfangstemperatur T_{D} gebracht und von einem sauerstoffhaltigen Gasgemisch bei Normaldruck durchströmt. Mit Erreichen von der Temperatur T_{D} wird eine 60 Gew.-%ige Lösung von 500 g 2,3,5-Trimethylphenol (**4**) in dem als Lösungsmittel dienenden Alkohol über einen Zeitraum t_{D} mit einer konstanten Rate zugeführt. Zur Vervollständigung der Reaktion wird für eine Zeitspanne t_{R} bei der Temperatur T_{R} nachgerührt.

Nach beendeter Reaktion und Abkühlung auf Raumtemperatur werden die Phasen getrennt, einzeln gewogen und die organische Phase analysiert. Der Umsatz von (**4**) ist in allen Fällen vollständig (>99,9%). Die Ausbeute an Chinon (**1**) variiert nur wenig und liegt in allen Versuchen im Bereich von 90-95%. Die Ergebnisse sind in Tabelle 1 aufgeführt.

Wie ein Vergleich der erfindungsgemäßen Beispiele (E 2, 4, 6, 8, 10) mit den zugehörigen Vergleichsbeispielen (C 1, 3, 5, 7, 9) zeigt, liegen die Gehalte an organisch gebundenem Chlor bei Durchführung der Reaktion in einem erfindungsgemäß zu verwendenden sekundären Alkohol im Mittel um den Faktor 3,5 niedriger als im Falle der Verwendung eines primären Alkohols. Der Gesamtchlor-, Kupfer- und Lithiumgehalt in der organischen Phase ist ebenfalls deutlich niedriger (im Mittel um den Faktor 6 für Gesamtchlor, um den Faktor 4,5 für Kupfer und um den Faktor 18 für Lithium).

**Tabelle 1: Ergebnisse der Oxidationsversuche (E: erfindungsgemäß, C: Vergleichsversuche).**

| Nr. | Lösungsmittel | t_{D}, h | T_{D}, °C | t_{R}, h | T_{R}, °C | O₂, NL/h | N₂, NL/h | Cl_{ges}, g/100g | Cl⁻, g/100g | Cl_{Organik}, g/100g | Cu, g/100g | Li, mg/kg |
|---|---|---|---|---|---|---|---|---|---|---|---|---|
| C1 | 1-Hexanol | 3.25 | 58 | 2.75 | 58 | 90 | 60 | 2.3 | 2.2 | 0.1 | 1.2 | 2100 |
| E2 | 3-Heptanol | 3.25 | 58 | 2.75 | 58 | 90 | 60 | 0.31 | 0.27 | 0.04 | 0.22 | 70 |
| C3 | 1-Hexanol | 2.00 | 53 | 4.00 | 65 | 90 | 60 | 2.5 | 2.3 | 0.2 | 1.1 | 2100 |
| E4 | 3-Heptanol | 2.00 | 53 | 4.00 | 65 | 90 | 60 | 0.31 | 0.26 | 0.05 | 0.21 | 65 |
| C5 | 1-Hexanol | 1.00 | 53 | 5.00 | 58 | 150 | 0 | 2.2 | 2.1 | 0.1 | 1.1 | 1800 |
| E6 | 3-Heptanol | 1.00 | 53 | 5.00 | 58 | 150 | 0 | 0.29 | 0.28 | 0.01 | 0.21 | 60 |
| E7 | 3-Heptanol | 1.00 | 53 | 5.00 | 1 h, 53 | 150 | 0 | 0.34 | 0.27 | 0.07 | 0.21 | 70 |
| | | | | | °C | | | | | | | |
| | | | | | 4 h 58 °C | | | | | | | |
| E8 | 2-Octanol | 1.00 | 53 | 5.00 | 58 | 150 | 0 | 0.47 | 0.42 | 0.05 | 0.29 | 230 |
| C9 | 1-Heptanol | 1.00 | 53 | 5.00 | 58 | 150 | 0 | 1.80 | 1.60 | 0.20 | 0.92 | 1500 |
| C1 0 | 1-Octanol | 1.00 | 53 | 5.00 | 58 | 150 | 0 | 1.70 | 1.50 | 0.20 | 0.83 | 1300 |

### Beispiel 11:

Ein aus Beispiel E7 erhaltener Reaktionsaustrag (Reaktion in 3-Heptanol) wird zunächst mit Wasser gewaschen. Nach erfolgter Phasentrennung wird die organische Phase mit wässriger HCl (25 Gew.-%) ausgeschüttelt und anschließend erneut mit Wasser gewaschen. Durch Zugabe von Natronlauge (2 Gew.-%) wird die Lösung auf pH=6 gebracht und das Lösungsmittel im Vakuum soweit entfernt, dass eine etwa 75 Gew.-%ige Lösung von Trimethylchinon (**1**) erhalten wird.

Zur Ermittlung der thermischen Stabilität dieses Rohproduktes wird dieses auf 110 °C erhitzt und in regelmäßigen Abständen der Gehalt von (**1**) durch Gaschromatographie bestimmt. Nach 125 Stunden hatten sich lediglich 8% des ursprünglich enthaltenen (**1**) zersetzt.

### Vergleichsbeispiel 12:

Beispiel 11 wurde mit dem Austrag aus Beispiel C1 (Reaktion in 1-Hexanol als Lösungsmittel) wiederholt. Beim Erhitzen auf 110°C waren nach 125 Stunden 44% des ursprünglich enthaltenen Chinons (**1**) zersetzt.

## Patentansprüche

1. Verfahren zur Herstellung von 2,3,5-Trimethylbenzochinon oder einer Mischung enthaltend 2,3,5-Trimethylbenzochinon umfassend den folgenden Schritt:
(i) Oxidation von 2,3,6-Trimethylphenol zu 2,3,5-Trimethylbenzochinon mit Sauerstoff oder einem sauerstoffhaltigen Gas in einem zwei- oder mehrphasigen Reaktionsmedium in Gegenwart eines Katalysators oder Katalysatorsystems mindestens enthaltend ein Kupfer(II)-halogenid, so dass eine Mischung enthaltend 2,3,5-Trimethylbenzochinon entsteht,
**dadurch gekennzeichnet, dass** das Reaktionsmedium Wasser und mindestens einen sekundären aliphatischen azyklischen Alkohol mit 6 oder mehr, vorzugsweise 7 oder mehr, Kohlenstoffatomen enthält.

2. Verfahren nach Anspruch 1, **dadurch gekennzeichnet, dass** der Katalysator oder das Katalysatorsystem Kupfer(II)-chlorid enthalt.

3. Verfahren nach mindestens einem der Ansprüche 1 bis 2 **dadurch gekennzeichnet, dass** der Katalysator oder das Katalysatorsystem weiterhin mindestens ein Alkalimetallhalogenid, vorzugsweise Lithiumchlorid, enthält.

4. Verfahren nach mindestens einem der Anspruche 1 bis 3 **dadurch gekennzeichnet, dass** das Reaktionsmedium 3-Heptanol enthält.

5. Verfahren nach mindestens einem der Ansprüche 1 bis 4 **dadurch gekennzeichnet, dass** das Verfahren diskontinuierlich durchgeführt wird.

6. Verfahren nach mindestens einem der Ansprüche 1 bis 5 **dadurch gekennzeichnet, dass** in einem Schritt (ii) die Mischung enthaltend 2,3,5-Trimethylbenzochinon mit einer wässrigen alkalischen Lösung gewaschen wird.

7. Verfahren nach mindestens einem der Ansprüche 1 bis 6, **dadurch gekennzeichnet, dass** die Oxidation bei einer Temperatur zwischen 50 °C und 65 °C, vorzugsweise bei einer Temperatur zwischen 53 und 58 °C durchgeführt wird.

8. Verfahren nach mindestens einem der Ansprüche 1 bis 7, **dadurch gekennzeichnet, dass** die Oxidation über einen Zeitraum von 4 bis 8 Stunden, vorzugsweise über einen Zeitraum von 4-7 Stunden, durchgeführt wird.

9. Verfahren nach mindestens einem der Ansprüche 1 bis 8 **dadurch gekennzeichnet, dass** das Reaktionsmedium nach erfolgter Oxidation in einem Schritt (iiI) einer Phasentrennung unterworfen und die organische Phase zur Rückgewinnung des in der organischen Phase gelösten Katalysators extrahiert wird.

## Claims

1. A process for preparing 2,3,5-trimethylbenzoquinone or a mixture comprising 2,3,5-trimethylbenzoquinone, comprising the following step:
(i) oxidizing 2,3,6-trimethylphenol to 2,3,5-trimethylbenzoquinone with oxygen or an oxygen-containing gas in a two-phase or multiphase reaction medium in the presence of a catalyst or catalyst system at least comprising a copper(II) halide, to give a mixture comprising 2,3,5-trimethylbenzoquinone,
wherein the reaction medium comprises water and at least one secondary aliphatic acyclic alcohol having 6 or more, preferably 7 or more, carbon atoms.

2. The process according to claim 1, wherein the catalyst or the catalyst system comprises copper(II) chloride.

3. The process according to at least one of claims 1 to 2, wherein the catalyst or the catalyst system further comprises at least one alkali metal halide, preferably lithium chloride.

4. The process according to at least one of claims 1 to 3, wherein the reaction medium comprises 3-heptanol.

5. The process according to at least one of claims 1 to 4, wherein the process is carried out batchwise.

6. The process according to at least one of claims 1 to 5, wherein the mixture comprising 2,3,5-trimethylbenzoquinone is washed in a step (ii) with an aqueous alkaline solution.

7. The process according to at least one of claims 1 to 6, wherein the oxidation is carried out at a temperature of between 50°C and 65°C, preferably at a temperature of between 53 and 58°C.

8. The process according to at least one of claims 1 to 7, wherein the oxidation is carried out over a period of 4 to 8 hours, preferably over a period of 4-7 hours.

9. The process according to at least one of claims 1 to 8, wherein the reaction medium, after oxidation has taken place, is subjected in a step (iii) to a phase separation, and the organic phase is extracted for recovery of the catalyst in solution in the organic phase.

## Revendications

1. Procédé pour la préparation de 2,3,5-triméthylbenzoquinone ou d'un mélange contenant de la 2,3,5-triméthylbenzoquinone, comprenant l'étape suivante :
(i) oxydation de 2,3,6-triméthylphénol en 2,3,5-triméthylbenzoquinone avec de l'oxygène ou un gaz contenant de l'oxygène dans un milieu de réaction à deux, ou plus, phases en présence d'un catalyseur ou d'un système catalytique contenant au moins un halogénure de cuivre (II), de sorte qu'un mélange contenant de la 2,3,5-triméthylbenzoquinone se forme,
**caractérisé en ce que** le milieu de réaction contient de l'eau et au moins un alcool acyclique aliphatique secondaire comprenant 6 ou plus, de préférence 7 ou plus, atomes de carbone.

2. Procédé selon la revendication 1, **caractérisé en ce que** le catalyseur ou le système catalytique contient du chlorure de cuivre (II).

3. Procédé selon au moins l'une quelconque des revendications 1 à 2, **caractérisé en ce que** le catalyseur ou le système catalytique contient en outre au moins un halogénure de métal alcalin, de préférence le chlorure de lithium.

4. Procédé selon au moins l'une quelconque des revendications 1 à 3, **caractérisé en ce que** le mélange réactionnel contient du 3-heptanol.

5. Procédé selon au moins l'une quelconque des revendications 1 à 4, **caractérisé en ce que** le procédé est réalisé de manière discontinue.

6. Procédé selon au moins l'une quelconque des revendications 1 à 5, **caractérisé en ce que**, dans une étape (ii), le mélange contenant de la 2,3,5-triméthylbenzoquinone est lavé avec une solution aqueuse alcaline.

7. Procédé selon au moins l'une quelconque des revendications 1 à 6, **caractérisé en ce que** l'oxydation est réalisée à une température entre 50°C et 65°C, de préférence à une température entre 53 et 58°C.

8. Procédé selon au moins l'une quelconque des revendications 1 à 7, **caractérisé en ce que** l'oxydation est réalisée sur une période de 4 à 8 heures, de préférence sur une période de 4-7 heures.

9. Procédé selon au moins l'une quelconque des revendications 1 à 8, **caractérisé en ce que** le milieu de réaction est soumis, après la fin de l'oxydation, dans une étape (iii), à une séparation des phases et la phase organique est extraite pour récupérer le catalyseur dissous dans la phase organique.
